(19) Europäisches Patentamt
European Patent Office
Office européen des brevets

(11) Publication number: 0 148 132 B1

(12) **EUROPEAN PATENT SPECIFICATION**

(45) Date of publication of patent specification: 06.09.89

(51) Int. Cl.$^4$: C 12 P 13/00 // (C12P13/00, C12R1:01, 1:19, 1:37, 1:42, 1:145)

(21) Application number: 84830296.4

(22) Date of filing: 31.10.84

# (54) Microbiological process for stereoselectively synthesizing L(-)-carnitine.

(30) Priority: 03.11.83 DD 256281

(43) Date of publication of application: 10.07.85 Bulletin 85/28

(45) Publication of the grant of the patent: 06.09.89 Bulletin 89/36

(84) Designated Contracting States: AT BE CH DE FR GB IT LI LU NL SE

(56) References cited:
EP-A-0 121 444
EP-A-0 122 794
EP-A-0 158 194
DE-A-2 454 048
GB-A-2 078 742
ACTA BIOL. MED. GERM., vol. 41, no. 11, 1982, pages 1009-1018; H. SEIM et al.: "Reduktiver Stoffwechsel des L-Carnitins und strukturverwandter Trimethylammoniumverbindungen in Escherichia coli"

(73) Proprietor: Sigma-Tau Industrie Farmaceutiche Riunite S.p.A.
47, Viale Shakespeare
I-00144 Rome (IT)

(72) Inventor: Seim, Hermann
Swiftstrasse 1B
D-7022 Leipzig (DD)
Inventor: Loester, Heinz
Wintergartenstrasse 2
D-7010 Leipzig (DD)
Inventor: Claus, Reiner
Leninstrasse 118
D-7050 Leipzig (DD)
Inventor: Kleber, Hans-Peter
Maxplanckstrasse 7
D-7010 Leipzig (DD)
Inventor: Strack, Erich
Phil. Rosenthalstrasse 7
D-7010 Leipzig (DD)

(74) Representative: Fassi, Aldo, Dr.
c/o Sigma-Tau Industrie Farmaceutiche Riunite S.p.A. Via Pontina, Km. 30, 400
I-00040 Pomezia (Roma) (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 148 132 B1

(56) References cited:
**CHEMICAL ABSTRACTS, vol. 97, no. 13, 27th September 1982, page 316, abstract no. 106821m, Columbus, Ohio, US; H. SEIM et al.: "Stimulation of the anaerobic growth of Salmonella typhimurium by reduction of L-carnitine, carnitine derivatives and structure-related trimethylammonium compounds", & ARCH. MICROBIOL. 1982, 132(1), 91-5**

**Description**

The present invention relates to a microbiological process for stereoselectively synthesizing L(−)-carnitine.

L(−)-carnitine (R(−) 3-hydroxy-4-trimethylaminobutyric acid) is normally present in the organism where it exerts the role of activated long-chain free fatty acids' carrier through the mitochondrial membrane. Since the mitochondrial membrane is impermeable to the acyl CoA's derivatives, the long-chain free fatty acids can cross it only when the esterification with L-carnitine has taken place. The importance of using L(−)-carnitine for therapeutical use has recently been underlined. ("Carnitine biosynthesis, metabolism, and functions" Editors: R. A. Frenkel and J. D. McGarry Academic Press 1980).

Up until recently, since sufficient amounts of L(−)-carnitine were not available, L(−)-carnitine was often substituted with the DL-carnitine racemate, obtainable by total chemical synthesis. But DL-carnitine, with contains the D(+), non physiological isomer, causes certain side-effects which do not take place using L(−)-carnitine (Curr. Ther. Res. *28* (1980), 195—198).

It is also known that the transferases necessary for the L(−)-carnitine formation, i.e. carnitine acetyl-transferase (EC 2.3.1.7) and carnitine palmitoyl-transferase (EC 2.3.1.21), are specific for the L(−) form, whereas the D(+) isomer is a competitive inhibitor of these two transferases. Administration of D(+)-carnitine can, moreover, bring about a decrease of L(−)-carnitine in the myocardium or in the skeletal muscle, as has been demonstrated on laboratory animals (guinea-pigs) (Life Sciences *28* (1981) 2931—2938). The symptoms of human hyperthyrosis which had improved after L(−)-carnitine treatment, worsened drastically with D(+)-carnitine (Endokrinologie, *38,* (1959) 218—225). Thus, for use in human therapy exclusively L(−)-carnitine must be used. This is also and especially valid in the case of patients having chronical kidney failure who have no possibility of actively eliminating the D(+) isomer.

Hemodialisis induces an L-carnitine secondary deficiency since it is eliminated in the dialysate, because of its low molecular weight (161.2). The oral administration or the addition of L(−)-carnitine to the dialysis solution avoids carnitine depletion in the patients.

In the case of hyperlipoproteinemia, a widespread condition in industrialised countries, a significative decrease in the levels of plasma risk-factors, triglycerides and cholersterol, has been achieved with L(−)-carnitine (Lancet II (1978) 805—807). Similar effects can be obtained using acyl-carnitines (DE—OS 2093579).

Several processes for obtaining L(−)-carnitine are already known.

L(−)-carnitine has been isolated from meat extracts via a complicated purification procedure.

In the '50's chemical syntheses were optimized though only DL-carnitine could be obtained.

Up till now, the isomer L(−) has been obtained via the racemate's resolution by means of fractional crystallisation of carnitine's salts with optically active acids. Various carnitine derivatives have been used, such as carnitine-nitrile, carnitinamide or even carnitine inner salt itself. As optically active acids, tartaric, camphoric acnd camphorsulphonic acids have been used (see, e.g., DD—PS 23217; DD—PS 93347; DE—OS 2927672).

DL-carnitine has also been resolved by means of specific L(−)-transferases.

However, since for the production of one mole of L(−)-carnitine, at least one mole of acyl CoA is needed, this process has turned out to be too expensive to apply industrially.

All other chemical syntheses with subsequent racemate solution have the further disadvantage that from synthesised DL-carnitine no more than 50% of the desired isomer can be isolated.

This problem has been overcome with enzymatic stereospecific synthesis starting from optically inactive precursors.

The US Patent 4,221,869 discloses the production of L(−)-carnitine from dehydrocarnitine with carnitine dehydrogenase (EC 1.1.1.108) isolated from Pseudomonas fluorescens, using NAD as coenzyme. Other enzymes are necessary, however, such as glucose dehydrogenase or alcohol dehydrogenase, for the regeneration of NADH. Moreover, dehydrocarnitine is very unstable and it spontaneously decomposes in acetonyltrimethylammonium and carbon dioxide.

The patent application DE—OS 3123975 dicloses L(−)-carnitine production from γ-butyrobetaine with γ-butyrobetaine hydroxylase (EC 1.14.11.1) isolated from "Neurospora crassa" mould. During this hydroxylation reaction, α-ketoglutaric acid and a reducing agent (e.g. ascorbic acid) must be added to the incubation medium. In order to achieve the maximum yield of L(−)-carnitine, catalase is also needed, γ-butyrobetaine hydroxylase is obtained after mould growth, isolation and purification of its spores with detergents and mechanical or ultrasonic treatment.

The precursors of the L(−)-carnitine biosynthesis are L-methionine and L-lysine. Through the ε-N,N,N trimethyl-lysine, ε-N,N,N trimethyl-β-hydroxy-lysine and N,N,N trimethylamino butyraldehyde intermediates, γ-butyrobetaine is formed. It is hydroxylated to L-carnitine by γ-butyrobetaines hydroxylase in the presence of molecular oxygen, α-ketoglutaric acid, ferrous ions and a reducing agent.

Crotonoylbetaine is not an intermediate in the biosynthesis. The European patent applications 0 121 444 (in the name of Hamari Yakuhin KKK; designating contracting states: CH, DE, FR, GB and LI) and 0 122 794 (in the name of Ajinomoto Co., Inc.; designated contracting states: DE, FR, IT and SE), both having priority dates earlier and publication dates later than the priority data of the present application disclose processes for producing L-carnitine by contacting crotonobetaine with a microorganism

belonging to the family Enterobacteriaceae containing the enzymes capable of stereoselectively hydrating crotonobetaine.

In the case of Hamari, their Enterobacter sp. microorganism has to be propagated in a very expensive complex culture medium while incubation is carried out at low concentrations of crotonobetaine (about 0.4%); these features make this method very costly and impractical for industrial production.

In the case of Ajinomoto, a wide variety of microorganisms, among which Citrobacter intermedius (IFO 13539), Escherichia coli (ATCC 10798), Hafnia alvei (ATCC 9760), Proteus mirabilis (ATCC 15290) and Salmonella gallinarum (ATCC 9184), are reported to catalyze the stereospecific hydration of crotonobetaine or salts thereof.

However, with a few exceptions, most of these microorganisms afford very low yields of L-carnitine which is unsuitable for industrial use.

The object of the present invention is to overcome the disadvantages of the known processes for L(−)-carnitine production and to provide a process that allows, in a favorably economical day, the biochemical production of the compound to be achieved.

The invention relates to a technically feasible process for producing L(−)-carnitine from easily available starting materials under simple reaction conditions and with simple intermediate separation techniques.

Until now it was only known that:

1. L(−)-carnitine, D(+)-carnitine and crotonoylbetaine are metabolized to γ-butyrobetaine.

2. The enterobacter growth stimulation depends on the γ-butyrobetaine formation, obtained by the crotonoylbetaine reduction (Arch. Microbiol. 132 (1982), 91—95).

3. The clostridia-bacteria are capable of reducing crotonic acid to butyric acid in anaerobic conditions. (FEBS Lett. *109,* (1980), 244—246).

Surprisingly it has now been found that even some bacteria, in certain particular conditions, are capable of stereospecifically hydroxylating crotonoylbetaine or a derivative thereof as will hereinbelow defined to L(−)-carnitine, a conversion which had not been observed in presence of γ-butyrobetaine in the incubation medium.

The microbiological process for stereoselectively synthesizing L(−)-carnitine according to the invention is characterized by contacting in a reaction medium an unsaturated compound selected from the group comprising crotonoylbetaine, crotonoylbetaine nitrile, salts, esters and amides of crotonoylbetaine, with a microorganism capable of stereoselectively hydroxylating said unsaturated compound to L(−)-carnitine, the microorganism being a member of at least one strain of the genera Escherichia, sp. Coli; Salmonella; Shigella; Citrobacter; Clostridium; Hafnia and Proteus.

Preferably, the strain of the genus Escherichia, sp. Coli, is selected from E. coli K12 Hfr. H; 044 K74; 055 K59; 0 111 K58 and 0 114 K90; the strain of the genus Salmonella is selected from S. typhimurium $LT_2$; S. cottbus; S. anatum and S. newington; the strain of the genus Shigella is S. flexneri 1a; the strain of the genus Citrobacter is C. freundii; the strain of the genus Clostridium is selected from C. sporogenes and C. kluyveri; the strain of the genus Hafnia is selected from H. alvei and H. alvei B; and the strain of the genus Proteus is selected from P. vulgaris and P. mirabilis.

To the bacteria growing on complex or minimal media or to the resting cells the unsaturated compound previously defined is added.

After a certain period of incubation the L(−)-carnitine formed is isolated from the reaction medium.

The crotonoylbetaine concentration in the incubation medium is comprised between 10 μ moles and 5 moles/l. As described in tables V, VI, VII the quantity of L(−)-carnitine formed increases with the increase of crotonoylbetaine concentration, but the percentual yield referred to the starting material decreases.

After the separation of the L(−)-carnitine from crotonoylbetaine the latter may be used again.

The bacteria capable of hydroxylating crotonoylbetaine can grow on the most different complex culture media, solid or liquid; thus, commercial nitritive media containing meat, yeast, malt and starch extracts can be used, adding, in partially anaerobic conditions, C and/or N sources such as ammonium hydroxide, urea, alcohol, carbohydrates, organic acids including fatty acids. The cultures are grown in calibrated containers in anaerobic condition and without stirring.

The resting cells are incubated in a minimal culture medium with salt solutions, organic and inorganic buffer mixtures which do not contain any C and/or N sources or whose C and N contents can not be used by the bacteria.

The incubation times are comprised between 3 hrs and 5 days. A preferred interval of time is comprised between 12 hrs and 48 hrs.

The reduction of crotonoylbetaine to γ-butyrobetaine leads to a decreased amount of converted L(−)-carnitine, thus this reduction reaction must be inhibited by adding electron acceptors of the aerobic or anaerobic respiration or hydrogen acceptors. Non-limiting examples of electron acceptors are oxygen, nitrates, trimethylamine N-oxide and other N-oxides, dimethyl sulphoxide, glucose, fructose, saccharose, maltose. Electron acceptors are fumarate, crotonate and acrylate. (Tables II, III, IV). The resting cells, previously grown on commercial nutrient media supplemented with meat broth or pancreatic peptone, are able to hydroxylate crotonoylbetaine to L(−)-carnitine (Table IX). The rate of L(−)-carnitine synthesis is increased, though, by inducing crotonoylbetaine hydroxylase formation, adding to the culture medium of growing bacteria crotonoylbetaine, DL-carnitine, fractions of DL-carnitine enriched with D(+) (obtained by

the chemical resolution of the racemate) or derivatives thereof (e.g. carboxylic esters, O-alkanoyl-carnitines or O-alkanoyl-carnitine esters). Crotonoylbetaine, its salts or its derivatives were prepared according to the following known processes:

1) Dehydration with sulphuric acid or acetic anhydride of DL-carnitine, D(+)-carnitine or D(+) enriched DL-carnitine, or alternatively elimination of the acid from the corresponding O-alkanoyl DL-carnitine or O-alkanoyl D-carnitine.

2) Exhaustive methylation of the 4-amino crotonate with methyl halogenides.

3) Reaction of trimethylamine with 4-halogen substituted crotonate.

A good separation of the L(−)-carnitine from unreacted crotonoylbetaine or from the formed γ-butyrobetaine, by ion-exchange cromatography ("Recent research on carnitine" Ed. G. Wolf, PG 11—21 (1965), US—PS 4,221,869; DE—OS 3123975) can only be carried out for small quantities. L(−)-carnitine in larger quantities has been separated after reaction of the hydroxy group withy the acyl chlorides of medium and long chain fatty acids (10—18 carbon atoms). The O-alkanoyl carnitines formed can be extracted from the water phase with n-butanol or isobutanol (Biochim. Biophys. Acta *280,* (1972), 422—433).

The O-alkanoyl carnitines are obtained by evaporation of the organic phase. They can be used directly or hydrolyzed with ammonium hydroxyde to L(−)-carnitine which is then utilized for therapeutical application.

The crotonoylbetaine which remains in the water phase is added back to the incubation medium.

Some of the advantages of this process are:

1) By dehydration of D(+)-carnitine or of D(+) enriched DL-carnitine, obtained by chemical resolution of the racemate, crotonoylbetaine, which is a cheap starting material for the L(−)-carnitine synthesis, is formed.

2) In this process no biochemical substances or other enzymes are to be employed.

3) The costs of incubation and of the starting materials are very low.

The following non-limiting examples illustrate the present invention.

Example 1

A 500 ml reaction vessel containing a complex culture medium with crotonoylbetaine:

pancreatic peptone 20 g/l of bidistilled $H_2O$

sodium chloride 5 g/l of bidistilled $H_2O$

crotonoylbetaine 50 m moles/l of bidistilled $H_2O$

pH 7 ($\Delta E_{540} = 0.050$)

was inoculated with a suspension of E. coli 044 K74, previously grown in 5% blood agar, in physiological solution.

The reaction flask was subsequently covered with paraffin oil and incubated at 37°C.

Table 1 shows the L(−)-carnitine formation in relation to the incubation time and the bacteria growth.

| Incubation time [h] | Growth [$\Delta E_{540}$] | L(−)-carnitine synthesis: m moles/l | L(−)-carnitine synthesis: $\frac{\text{L(−)-carnitine moles}}{\text{crotonobetaine moles}} \times 10^2$ |
|---|---|---|---|
| 0 | 0.0 | - | - |
| 3 | 0.100 | - | - |
| 6 | 0.120 | 0.5 | 0.9 |
| 12 | 0.190 | 2.0 | 3.9 |
| 24 | 0.200 | 6.7 | 13.4 |
| 48 | 0.175 | 6.6 | 13.3 |

Example 2

The complex culture medium containing crotonoylbetaine, inoculated with E. coli (example 1), after 24 hrs of incubation was centrifuged at 6000 rounds to separate the grown bacteria. The obtained centrifugate was washed with a Sorensen phosphate buffer 0.01 M at pH 7.5 (0.20 g/l $KH_2PO_4$ and 3.05 g/l $Na_2HPO_5 \cdot 12 H_2O$), suspended in a 1 l Sorensen phosphate buffer containing 5 g/l crotonoylbetaine (34.9 m moles/l) ($\Delta E_{540} = 0.080$) and maintained for 24 hrs at 37°C. Subsequently it was again centrifuged and 9.08 m moles/l of L(−)-carnitine were found in the solution. Yield: 26% in relation to crotonoylbetaine.

Example 3

To a complex culture medium containing L(−)-carnitine (31 m moles/l of L-carnitine corresponding to 5

g/l) and inoculated, as in example 1, with several strains of enterobacteria, electron acceptors of the anaerobic respiration and other substances (sodium succinate, sodium fumarate and glucose 10 g/l, potassium nitrate, trimethylamine N-oxide (TMO) 5 g/l) were added.

Table II illustrates the yield in moles of γ-butyrobetaine in relation with the L(−)-carnitine utilized.

| Bacteria strains | γ-butyrobetaine formation [% moles] | | | | | |
|---|---|---|---|---|---|---|
| | - | succinate | fumarate | $KNO_3$ | TMO | glucose |
| Escherichia coli 044 K74 | 71 | 77 | 13 | 0 | 0 | 0 |
| Escherichia coli K12 Hfr H | 45 | 43 | 5 | 0 | 0 | 0 |
| Salmonella typhimurium $LT_2$ | 87 | 89 | 68 | 0 | 8 | 71 |
| Proteus vulgaris | 79 | 81 | 17 | 0 | 17 | 0 |

Example 4

To the complex culture medium (KM) described in example 1, or to the minimal culture medium (MM) consisting of:

| | |
|---|---|
| $Na_2HPO_4 \cdot 12H_2O$ | 15.0 g |
| $KH_2PO_4$ | 3.0 g |
| $NH_4Cl$ | 1.0 g |
| $MgSO_4 \cdot H_2O$ | 0.15 g |
| $CaCl_2$ | 0.014 g |
| $FeCl_3$ | 0.0002 g |
| D-ribose | 7.5 g |

distilled $H_2O$ sufficient for 1 l

50 m moles of crotonoylbetaine and sodium fumarate (10 g/l), potassium nitrate (5 g/l), and trimethylamine N-oxide (5 g/l) were added. The media were then incubated with a layer of paraffin (anaerobic conditions) or without paraffin (aerobic conditions), at 37°C with E. coli 044 K74 for 48 hrs.

Table III : Influence of electron acceptors on the L-carnitine formation in the growing bacteria

| Culture medium | L(−)-carnitine synthesis m moles/l | | | | |
|---|---|---|---|---|---|
| | $O_2$ absence | $O_2$ presence | fumarate | $KNO_3$ | TMO |
| KM | 4.5 | 2.6 | 10.7 | 0.9 | 5.1 |
| MM | 2.7 | 1.5 | 10.8 | 0 | 5.9 |

Example 5

A complex culture medium (KM) or a minimal culture medium (MM) containing 50 m moles/l of crotonoylbetaine were inoculated with E. coli 044 K74 and incubated for 48 hrs. The cells, collected and washed twice (example 2), were incubated for 48 hrs in Sorensen buffer containing 50 m moles/l of crotonoylbetaine to which the electron acceptors were added as in example 4.

Table IV : Influence of the electron acceptors on the L-carnitine formation with resting cells

| Culture medium | L(-)-carnitine synthesis m moles/l | | | | |
|---|---|---|---|---|---|
| | $O_2$ absence | $O_2$ presence | fumarate | $KNO_3$ | TMO |
| KM | 7.5 | 8.3 | 0 | 7.8 | 8.4 |
| MM | 9.1 | 12.1 | 0 | 9.8 | 10.4 |

Example 6

Minimal culture media (MM), with various crotonoylbetaine concentrations, supplemented with D-ribose as C source (example 4) were inoculated with E. coli 044 K74 (example 1) and incubated at 37°C for 48 hrs.

| crotonoyl-betaine [m moles/l] | L-carnitine synthesis [m moles/l] | $\frac{\text{L-carnitine moles}}{\text{crotonoylbetaine moles}} \times 10^2$ | Growth [$E_{540}$] |
|---|---|---|---|
| 500 | 5.3 | 1.1 | 0.120 |
| 50 | 4.3 | 8.6 | 0.450 |
| 5 | 0.3 | 6.0 | 0.385 |
| 0.5 | 0.09 | 18.0 | 0.220 |

Example 7

A complex culture medium as described in example 1 (A) and a minimal culture medium supplemented with D-ribose, as described in example 4 (B), each containing 8 g/l of L(−)-carnitine, were inoculated with E. coli 044 K74. After 48 hrs the collected bacteria, washed twice, were suspended in Sorensen buffer, containing various crotonoylbetaine concentrations and incubated for 48 hrs at 37°C.

Table VI: L(−)-carnitine synthesis with induced resting cells by L(−)-carnitine at various crotonoylbetaine concentrations

| crotonoylbetaine [m moles/l] | L-carnitine synthesis | |
|---|---|---|
| | [m moles/l] | L-carnitine moles / crotonoylbetaine moles x10[2] |
| A) 500 | 9.0 | 1.8 |
| 50 | 7.5 | 15.1 |
| 5 | 2.9 | 58.4 |
| B) 500 | 16.2 | 3.2 |
| 50 | 8.3 | 16.5 |
| 5 | 3.7 | 74.0 |

Example 8

A complex culture medium containing crotonoylbetaine as in example 1 inoculated with E. coli 044 K74 was centrifuged after 48 hrs. The centrifugate, washed twice, was dispersed in a minimal culture medium, without C and N sources, containing various concentrations of crotonoylbetaine and incubated for 48 hrs.

Table VII: L-carnitine synthesis with resting cells induced by crotonoylbetaine at various crotonoylbetaine concentrations

| crotonoylbetaine [m moles/l] | L-carnitine synthesis | |
|---|---|---|
| | [m moles/l] | L-carnitine moles / crotonoylbetaine moles x10[2] |
| 500 | 24.3 | 4.9 |
| 50 | 12.2 | 24.5 |
| 5 | 2.4 | 48.8 |
| 0.5 | 0.47 | 94.0 |

Example 9

A minimal culture medium as described in example 4 which contains, instead of crotonoylbetaine, 50 m moles/l of L(−)-carnitine was inoculated with E. coli 044 K74 and incubated for 48 hrs. Subsequently the collected and twice washed bacteria were incubated at 37°C in a minimal culture medium without C and N sources but with 50 m moles/l of crotonoylbetaine. The optical density at the beginning of the incubation was $\Delta E_{540} = 0.210$.

Table VIII: L(-)-carnitine formation with resting cells in function of time

| Incubation time [h] | L-carnitine synthesis [moles/l] | L(-)-carnitine moles / crotonoylbetaine moles x10$^2$ |
|---|---|---|
| 3 | 2.5 | 5.1 |
| 6 | 3.7 | 7.5 |
| 12 | 6.6 | 13.2 |
| 24 | 9.0 | 18.0 |
| 48 | 10.1 | 20.3 |

Example 10

A complex culture medium (KM) or a minimal culture medium (MM) without C sources, supplemented with the substances indicated in Table IX, at the concentrations of 50 m moles/l, was inoculated with E. coli 044 K74 and incubated for 48 hrs. Subsequently, the collected bacteria, washed twice with Sorensen buffer, were incubated for 48 hrs in Sorensen buffer with 50 m moles/l crotonoylbetaine.

Table IX: L-carnitine formation with resting cells in function of the various substances' addition in the growth medium

| | Growing cells | | Resting cells | |
|---|---|---|---|---|
| Medium | added substances | $\Delta E_{540}$ after 48 hrs | $\Delta E_{540}$ at the baginning | L-carnitine synthesis m moles/l |
| KM | - | 0.315 | 0.225 | 1.4 |
| MM | D-Ribose | 0.196 | 0.136 | 0.3 |
| MM | D-Glucose | 0.325 | 0.260 | 0.6 |
| MM | D-Ribose Fumarate | 0.165 | 0.100 | 0 |
| MM | D-Ribose, GABOB+ | 0.395 | 0.220 | 0.3 |
| MM | D-Ribose L-carnitine | 0.255 | 0.190 | 6.6 |

+DL-$\gamma$ amino $\beta$-hydroxybutyric acid.

**Claims for the Contracting States: AT BE LU NL**

1. A microbiological process for producing L(−)-carnitine characterized in that it comprises:
(a) preparing a bacterial culture medium comprising an unsaturated compound selected from crotonobetaine, a crotonobetaine derivative;
(b) inoculating the culture medium with a microorganism capable of stereoselectively hydrating said unsaturated compound to L(−)-carnitine;
(c) incubating said culture medium; and
(d) recovering L(−)-carnitine from the culture medium.

2. The process of claim 1 characterized in that the microorganism is a member of at least one strain of the genera Escherichia, sp. Coli; Salmonella; Shigella; Citrobacter; Clostridium; Hafnia and Proteus.

3. The process of claim 2, characterized in that the strain of the genus Escherichia, sp. Coli, is selected from E. Coli 044 K74; 055 K59; 0111 K58 and 0114 K90.

4. The process of claim 2, characterized in that the strain of the genus Salmonella is selected from S. typhimurium LT2; S. cottbus; S. anatum and S. newington.

5. The process of claim 2, characterized in that the strain of the genus Shigella is S. flexneri 1a;

6. The process of claim 2, characterized in that the strain of the genus Citrobacter is C. freundii.

7. The process of claim 2, characterized in that the strain of the genus Clostridium is selected from C. sporogenes and C. Kluyveri.

8. The process of claim 2, characterized in that the strain of the genus Hafnia is selected from H. alvei A and H. alvei B.

9. The process of claim 2, characterized in that the strain of the genus Proteus is selected from P. vulgaris and P. mirabilis.

10. The process of claims 1—9 characterized in that the crotonobetaine derivative is selected from the group consisting of crotonobetaine salts, crotonobetaine nitrile, crotonobetaine amides, and crotonobetaine esters.

11. The process of claims 1—10 characterized in that the concentration of the unsaturated compound in the culture medium is comprised between 10 μ moles and 5 moles/liter.

12. The process of claims 1—11 characterized in that the culture medium is selected from a complex culture medium, a minimal culture medium and a pH 7—8 buffered aqueous solution containing said unsaturated compound as the only carbon and nitrogen source capable of being metabolized by said microorganism.

13. The process of claim 12, characterized in that the reaction medium comprises an inhibitor of the reduction of the unsaturated compound to the corresponding saturated compound, selected from electron acceptors of the aerobic or anaerobic respiration and hydrogen acceptors.

14. The process of claim 13, characterized in that said inhibitor is selected from the group comprising nitrates, trimethylamine N-oxide and other N-oxides, glucose, fructose, saccharose, maltose, dimethylsulfoxide, fumarate, crotonate and acrylate.

15. The process of claims 1—14, characterized in that the microorganism is a growing microorganism.

16. The process of claim 1—14, characterized in that the microorganism is a resting microorganism.

17. The process of claim 15, characterized in that the microorganism has been previously subjected to induction.

18. The process of claim 17, characterized in that the induction has been carried out by adding to the culture medium an inducing agent selected from crotonoylbetaine L(−), (D+), DL-carnitine, carboxyl esters, O-alkanoyl derivatives and esters of O-alkanoyl derivatives of (L−), (D+), DL-carnitine and salts thereof.

19. The process of claim 1—18, characterized in that separation of the obtained L(−)-carnitine from the unreacted crotonoylbetaine takes place by converting L(−)-carnitine into an O-alkanoyl carnitine wherein the alkanoyl group has from 10 to 18 carbon atoms and extracting from an aqueous phase containing O-alkanoyl carnitine and crotonoylbetaine the O-alkanoyl carnitine in a water immiscible alkanol selected from n-butanol and isobutanol.

20. The process of claim 1—19 characterized in that the incubation takes place in anaerobic conditions.

**Claims for the Contracting States: CH GB LI**

1. A microbiological process for producing L(−)-carnitine which comprises:

(a) preparing a bacterial culture medium comprising an unsaturated compound selected from crotonobetaine, a crotonobetaine derivative;

(b) inoculating the culture medium with a microorganism capable of stereoselectively hydrating said unsaturated compound to L(−)-carnitine;

(c) incubating said culture medium; and

(d) recovering L(−)-carnitine from the culture medium, characterized in that said microorganism is selected from the group consisting of:

Escherichia Coli 044 K 74
Escherichia Coli 055 K 59
Escherichia Coli 0111 K 58
Escherichia Coli 0114 K 90
Salmonella typhimurium Lt2
Salmonella cottbus
Salmonella anatum
Salmonella newington
Shigella flexneri
Clostridium kluyveri
Clostridium sporogenes
Citrobacter freundii
Proteus vulgaris
Proteus mirabilis
Hafnia alvei.

2. The process of claim 1 characterized in that the crotonobetaine derivative is selected from the group consisting of crotonobetaine nitrile, crotonobetaine amides, and crotonobetaine esters.

3. The process of claims 1 and 2 characterized in that the concentration of the unsaturated compound in the culture medium is comprised between 10 μ moles and 5 moles/liter.

4. The process of claims 1—3 characterized in that the culture medium is selected from a complex culture medium, a minimal culture medium and a pH 7—8 buffered aqueous solution containing said unsaturated compound as the only carbon and nitrogen source capable of being metabolized by said microorganism.

5. The process of claim 4, characterized in that the reaction medium comprises an inhibitor of the reduction of the unsaturated compound to the corresponding saturated compound, selected from electron acceptors of the aerobic or anaerobic respiration and hydrogen acceptors.

6. The process of claim 5, characterized in that said inhibitor is selected from the group comprising nitrates, trimethylamine N-oxide and other N-oxides, glucose, fructose, saccharose, maltose, dimethylsulfoxide, fumarate, crotonate and acrylate.

7. The process of claims 1—6, characterized in that the microorganism is a growing microorganism.

8. The process of claims 1—6, characterized in that the microorganism is a resting microorganism.

9. The process of claim 7, characterized in that the microorganism has been previously subjected to induction.

10. The process of claim 9, characterized in that the induction has been carried out by adding to the culture medium an inducing agent selected from crotonoylbetaine, L(−), (D+), DL(−)-carnitine, carboxyl esters, O-alkanoyl derivatives and esters of O-alkanoyl derivatives of (L−), (D+), DL-carnitine and salts thereof.

11. The process of claims 1—10, characterized in that separation of the obtained L(−)-carnitine from the unreacted crotonoylbetaine takes place by converting L(−)-carnitine into an O-alkanoyl carnitine wherein the alkanol group has from 10 to 18 carbon atoms and extracting from an aqueous phase containing O-alkanoyl carnitine and crotonoylbetaine the O-alkanoyl carnitine in a water immiscible alkanol selected from n-butanol and isobutanol.

12. The process of claim 1—11 characterized in that the incubation takes place in anaerobic conditions.

**Claims for the Contracting States: DE FR IT SE**

1. A microbiological process for producing L(−)-carnitine which comprises:

(a) preparing a bacterial culture medium comprising an unsaturated compound selected from crotonobetaine, a crotonobetaine derivative;

(b) inoculating the culture medium with a microorganism capable of stereoselectively hydrating said unsaturated compound to L(−)-carnitine;

(c) incubating said culture medium; and

(d) recovering L(−)-carnitine from the culture medium, characterized in that said microorganism is selected from the group consisting of:

Escherichia Coli 044 K 74
Escherichia Coli 055 K 59
Escherichia Coli 0111 K 58
Escherichia Coli 0114 K 90
Salmonella typhimurium Lt2
Salmonella cottbus
Salmonella anatum
Salmonella newington
Shigella flexneri
Clostridium kluyveri
Clostridium sporogenes
Citrobacter freundii
Proteus vulgaris

2. The process of claim 1 characterized in that the crotonobetaine derivative is selected from the group consisting of crotonobetaine nitrile, crotonobetaine amides, and crotonobetaine esters.

3. The process of claims 1 and 2 characterized in that the concentration of the unsaturated compound in the culture medium is comprised between 10 μ moles and 5 moles/liter.

4. The process of claims 1—3 characterized in that the culture medium is selected from a complex culture medium, a minimal culture medium and a pH 7—7 buffered aqueous solution containing said unsaturated compound as the only carbon and nitrogen source capable of being metabolized by said microorganism.

5. The process of claim 4, characterized in that the reaction medium comprises an inhibitor of the reduction of the unsaturated compound to the corresponding saturated compound, selected from electron acceptors of the aerobic or anaerobic respiration and hydrogen acceptors.

6. The process of claim 5, characterized in that said inhibitor is selected from the group comprising nitrates, trimethylamine N-oxide and other N-oxides, glucose, fructose, saccharose, maltose, dimethylsulfoxide, fumarate, crotonate and acrylate.

7. The process of claims 1—6, characterized in that the microorganism is a growing microorganism.

8. The process of claims 1—6, characterized in that the microorganism is a resting microorganism.

9. The process of claim 7, characterized in that the microorganism has been previously subjected to induction.

10. The process of claim 9, characterized in that the induction has been carried out by adding to the culture medium an inducing agent selected from crotonoylbetaine, L(−), (D+), L(−)-carnitine, carboxyl esters, O-alkanoyl derivatives and esters of O-alkanoyl derivatives of (L−), (D+), DL-carnitine and salts thereof.

11. The process of claims 1—10, characterized in that separation of the obtained L(−)-carnitine from the unreacted crotonoylbetaine takes place by converting L(−)-carnitine into an O-alkanoyl carnitine wherein the alkanol group has from 10 to 18 carbon atoms and extracting from an aqueous phase containing O-alkanoyl carnitine and crotonoylbetaine the O-alkanoyl carnitine in a water immiscible alkanol selected from n-butanol and isobutanol.

12. The process of claim 1—11 characterized in that the incubation takes place in anaerobic conditions.

**Patentansprüche für die Vertragsstaaten: AT BE LU NL**

1. Mikrobioligisches Verfahren zur Herstellung von L(−)-Carnitin, welches umfaßt:

(a) Herstellung eines Bakterien-Kulturmediums, das eine ungesättigte Verbindung, ausgewählt aus Crotonbetain, einem Crotonbetainderivat, enthält;

(b) Beimpfen des Kulturmediums mit einem Mikroorganismus, der zur stereoselektiven Hydrierung der ungesättigten Verbindung zu L(−)-Carnitin geeignet ist;

(c) Inkubation des Mikroorganismus; und

(d) Gewinnung von L(−)-Carnitin aus dem Kulturmedium.

2. Verfahren nach Anspruch 1, dadurch gekennzeichnet, daß der Mikroorganismus ein Vertreter mindestens eines Stammes der Gattungen Escherichia, sp.Coli; Salmonella; Shigella; Citrobacter; Clostridium, Hafnia und Proteus ist.

3. Verfahren nach Anspruch 2, dadurch gekennzeichnet, daß der Stamm der Gattung Escherichia, sp.Coli aus E. Coli 044 K74; 055 K59; 0111 K58 und 0114 K90 ausgewählt ist.

4. Verfahren nach Anspruch 2, dadurch gekennzeichnet, daß der Stamm der Gattung Salmonella aus S. typhimurium LT2; S. cottbus; S. anatum und S. newington ausgewählt ist.

5. Verfahren nach Anspruch 2, dadurch gekennzeichnet, daß der Stamm der Gattung Shigella S. flexneri la ist.

6. Verfahren nach Anspruch 2, dadurch gekennzeichnet, daß der Stamm der Gattung Citrobacter C. freundii ist.

7. Verfahren nach Anspruch 2, dadurch gekennzeichnet, daß der Stamm der Gattung Clostridium aus C. sporogenes und C. Kluyveri ausgewählt wird.

8. Verfahren nach Anspruch 2, dadurch gekennzeichnet, daß der Stamm der Gattung Hafnia aus H. alvei A und H. Alvei B ausgewählt wird.

9. Verfahren nach Anspruch 2, dadurch gekennzeichnet, daß der Stamm der Gattung Proteus aus P. vulgaris und P. mirabilis ausgewählt wird.

10. Verfahren nach den Ansprüchen 1—9, dadurch gekennzeichnet, daß das Crotonbetainderivat aus der Gruppe bestehend aus Crotonbetainsalzen, Crotonbetainnitril, Crotonbetainamiden und Crotonbetainestern oder Mischungen davon, ausgewählt wird.

11. Verfahren nach den Ansprüchen 1—10, dadurch gekennzeichnet, daß die Konzentration der ungesättigten Verbindung in dem Kulturmedium zwischen 10 µMol und 5 Mol/1 liegt.

12. Verfahren nach den Ansprüchen 1—11, dadurch gekennzeichnet, daß das Kulturmedium aus einem komplexen Kulturmedium, einem minimalen Kulturmedium und einer auf pH 7—8 gepufferten wässerigen Löbsung, welche die ungesättigte Verbindung als einzige durch den Mikroorganismus metabolisierbarem Kohlenstoff- und Stickstoffquelle enthält, ausgewählt wird.

13. Verfahren nach Anspruch 12, dadurch gekennzeichnet, daß das Reaktionsmedium einen Inhibitor der Reduktion der ungesättigten Verbindung zur entsprechenden gesättigten Verbindung enthält, ausgewählt aud Elektronenakzeptoren der aeroben oder anaeroben Atmung und Wasserstoffakzeptoren.

14. Verfahren nach Anspruch 13, dadurch gekennzeichnet, daß der Inhibitor aus der Gruppe bestehend aus Nitraten, Trimethylamin-N-oxid und anderen N-Oxiden, Glucose, Fructose, Saccharose, Maltose, Dimethylsulfoxid, Fumarat, Crotonat und Acrylat ausgewählt ist.

15. Verfahren nach den Ansprüchen 1—14, dadurch gekennzeichnet, daß der Mikroorganismus ein wachsender Mikroorganismus ist.

16. Verfahren nach den Ansprüchen 1—14, dadurch gekennzeichnet, daß der Mikroorganismus ein ruhender Mikroorganismus ist.

17. Verfahren nach Anspruch 15, dadurch gekennzeichnet, daß der Mikroorganismus zuvor einer Induktion unterworfen worden ist.

18. Verfahren nach Anspruch 17, dadurch gekennzeichnet, daß die Induktion durch Zusatz eines induzierenden Agens, ausgewählt aus Grotonoylbetain, L(−), (D+), DL-Carnitin, Carboxylestern, O-Alkanoylderivaten und Estern von O-Alkanoylderivaten von (L−), (D+), DL-Carnitin und Salzen davon, zum Kulturmedium ausgeführt wird.

19. Verfahren nach den Ansprüchen 1—18, dadurch gekennzeichnet, daß die Trennung des erhaltenen (L-)-Carnitins von nichtumgesetzten Crotonoylbetain durch Umwandlung von (L−)-Carnitin in eine O-Alkanoylcarnitin, worin die Alkanoylgruppe 10 bis 18 Kohlenstoffatome hat, und Extrahieren des O-Alkanoylcarnitins aus einer wässerigen Phase, die O-Alkanoylcarnitin und Crotonoylbetain enthält, in einen mit Wasser nichtmischbaren Alkohol, ausgewählt aus N-Butanol und isobutanol, erfolgt.

20. Verfahren nach den Ansprüchen 1—19, dadurch gekennzeichnet, daß die Inkubation unter anaeroben Bedingungen erfolgt.

**Patentansprüche für die Vertragsstaaten: CH GB LI**

1. Mikrobioligisches Verfahren zur Herstellung von L(−)-Carnitin, welches umfaßt:

(a) Herstellung eines Bakterien-Kulturmediums, das eine ungesättigte Verbindung, ausgewählt aus

Crotonbetain, einem Crotonbetainderivat, enthält;

(b) Beimpfen des Kulturmediums mit einem Mikroorganismus, der zur stereoselektiven Hydrierung der ungesättigten Verbindung zu L(−)-Carnitin geeignet ist;

(c) Inkubation des Mikroorganismus; und

(d) Gewinnung von L(−)-Carnitin aus dem Kulturmedium, dadurch gekennzeichnet, daß der Mikroorganismus aus der Gruppe bestehend aus:

Escherichia Coli 044 K 74
Escherichia Coli 055 K 59
Escherichia Coli 0111 K 58
Escherichia Coli 0114 K 90
Salmonella typhimurium Lt2
Salmonella cottbus
Salmonella anatum
Salmonella newington
Shigella flexneri
Clostridium kluyveri
Clostridium sporogenes
Citrobacter freundii
Proteus vulgaris
Proteus mirabilis
Hafnia alvei

ausgewählt wird.

2. Verfahren nach Anspruch 1, dadurch gekennzeichnet, daß das Crotonbetainderivat aus der Gruppe bestehend aus Crotonbetainnitril, Crotonbetainamid, Aryl- und Alkylcrotonbetainestern oder Mischungen davon, aus gewählt wird.

3. Verfahren nach Anspruch 1 und 2, dadurch gekennzeichnet, daß die Konzentration der ungesättigten Verbindung in dem Kulturmedium zwischen 10 µMol und 5 Mol/1 liegt.

4. Verfahren nach den Ansprüchen 1—3, dadurch gekennzeichnet, daß das Kulturmedium aus einem komplexen Kulturmedium, einem minimalen Kulturmedium und einer auf pH 7—8 gepufferten wässerigen Lösung, welche die ungesättigte Verbindung als einzige durch den Mikroorganismus metabolisierbarem Kohlenstoff- und Stickstoffquelle enthält, ausgewählt wird.

5. Verfahren nach Anspruch 4, dadurch gekennzeichnet, daß das Reaktionsmedium einen Inhibitor der Reduktion der ungesättigten Verbindung zur entsprechenden gesät tigten Verbindung enthält, ausgewählt aus Elektronenakzeptoren der aeroben oder anaeroben Atmung und Wasserstoffakzeptoren.

6. Verfahren nach Anspruch 5, dadurch gekennzeichnet, daß der Inhibitor aus der Gruppe bestehend aus Nitraten, Trimethylamin-N-oxid und anderen N-Oxiden, Glucose, Fructose, Saccharose, Maltose, Dimethylsulfoxid, Fumarat, Crotonat und Acrylat ausgewählt ist.

7. Verfahren nach den Ansprüchen 1—6, dadurch gekennzeichnet, daß der Mikroorganismus ein wachsender Mikroorganismus ist.

8. Verfahren nach den Ansprüchen 1—6, dadurch gekennzeichnet, daß der Mikroorganismus ein ruhender Mikroorganismus ist.

9. Verfahren nach Anspruch 7, dadurch gekennzeichnet, daß der Mikroorganismus zuvor einer Induktion unterworfen worden ist.

10. Verfahren nach Anspruch 9, dadurch gekennzeichnet, daß die Induktion durch Zusatz eines induzierenden Agens, ausgewählt aus Crotonoylbetain, L(−), (D+), DL-Carnitin, Carboxylestern, O-Alkanoylderivaten und Estern von O-Alkanoylderivaten von (L−), (D+), DL-Carnitin und Salzen davon, zum Kulturmedium ausgeführt wird.

11. Verfahren nach den Ansprüchen 1—10, dadurch gekennzeichnet, daß die Trennung des erhaltenen (L-)-Carnitins von nichtumgesetzten Crotonoylbetain durch Umwandlung von (L−)-Carnitin in ein O-Alkanoylcarnitin, worin die Alkanoylgruppe 10 bis 18 Kohlenstoffatome hat, und Extrahieren des O-Alkanoylcarnitins aus einer wässerigen Phase, die O-Alkanoylcarnitin und Crotonoylbetain enthält, in einen mit Wasser nichtmischbaren Alkohol, ausgewählt aus N-Butanol und isobutanol, erfolgt.

12. Verfahren nach den Ansprüchen 1—11, dadurch gekennzeichnet, daß die Inkubation unter anaeroben Bedingungen erfolgt.

**Patentansprüche für die Vertragsstaaten: DE FR IT SE**

1. Mikrobioligisches Verfahren zur Herstellung von L(−)-Carnitin, welches umfaßt:

(a) Herstellung eines Bakterien-Kulturmediums, das eine ungesättigte Verbindung, ausgewählt aus Crotonbetain, einem Crotonbetainderivat, enthält;

(b) Beimpfen des Kulturmediums mit einem Mikroorganismus, der zur stereoselektiven Hydrierung der ungesättigten Verbindung zu L(−)-Carnitin geeignet ist;

(c) Inkubation des Mikroorganismus; und

(d) Gewinnung von L(−)-Carnitin aus dem Kulturmedium, dadurch gekennzeichnet, daß der Mikroorganismus aus der Gruppe bestehend aus:

Escherichia Coli 044 K 74
Escherichia Coli 055 K 59
Escherichia Coli 0111 K 58
Escherichia Coli 0114 K 90
Salmonella typhimurium Lt2
Salmonella cottbus
Salmonella anatum
Salmonella newington
Shigella flexneri
Clostridium kluyveri
Clostridium sporogenes
Citrobacter freundii
Proteus vulgaris
ausgewählt wird.

2. Verfahren nach Anspruch 1, dadurch gekennzeichnet, daß das Crotonbetainderivat aus der Gruppe bestehend aus Crotonbetainnitril, Crotonbetainamid den und Croton betainestern ausgewählt wird.

3. Verfahren nach Anspruch 1 und 2, dadurch gekennzeichnet, daß die Konzentration der ungesättigten Verbindung in dem Kulturmedium zwischen 10 µMol und 5 Mol/1 liegt.

4. Verfahren nach den Ansprüchen 1—3, dadurch gekennzeichnet, daß das Kulturmedium aus einem komplexen Kulturmedium, einem minimalen Kulturmedium und einer auf pH 7—8 gepufferten wässerigen Löbsung, welche die ungesättigte Verbindung als einzige durch den Mikroorganismus metabolisierbarem Kohlenstoff- und Stickstoffquelle enthält, ausgewählt wird.

5. Verfahren nach Anspruch 4, dadurch gekennzeichnet, daß das Reaktionsmedium einen Inhibitor der Reduktion der ungesättigten Verbindung zur entsprechenden gesättigten Verbindung enthält, ausgewählt aus Elektronenakzeptoren der aeroben oder anaeroben Atmung und Wasserstoffakzeptoren.

6. Verfahren nach Anspruch 5, dadurch gekennzeichnet, daß der Inhibitor aus der Gruppe bestehend aus Nitraten, Trimethylamin-N-oxid und anderen N-Oxiden, Glucose, Fructose, Saccharose, Maltose, Dimethylsulfoxid, Fumarat, Crotonat und Acrylat ausgewählt ist.

7. Verfahren nach den Ansprüchen 1—6, dadurch gekennzeichnet, daß der Mikroorganismus ein wachsender Mikroorganismus ist.

8. Verfahren nach den Ansprüchen 1—6, dadurch gekennzeichnet, daß der Mikroorganismus ein ruhender Mikroorganismus ist.

9. Verfahren nach Anspruch 7, dadurch gekennzeichnet, daß der Mikroorganismus zuvor einer Induktion unterworfen worden ist.

10. Verfahren nach Anspruch 9, dadurch gekennzeichnet, daß die Induktion durch Zusatz eines induzierenden Agens, ausgewählt aus Crotonoylbetain, L(−), (D+), DL-Carnitin, Carboxylestern, O-Alkanoylderivaten und Estern von O-Alkanoylderivaten von (L−), (D+), DL-CArnitin und Salzen davon, zum Kulturmedium ausgeführt wird.

11. Verfahren nach den Ansprüchen 1—10, dadurch gekennzeichnet, daß die Trennung des erhaltenen (L-)-Carnitins von nichtumgesetzten Crotonoylbetain durch Umwandlung von (L−)-Carnitin in ein O-Alkanoylcarnitin, worin die Alkanoylgruppe 10 bis 18 Kohlenstoffatome hat, und Extrahieren des O-Alkanoylcarnitins aus einer wässerigen Phase, die O-Alkanoylcarnitin und Crotonoylbetain enthält, in einen mit Wasser nichtmischbaren Alkohol, ausgewählt aus n-Butanol und isobutanol, erfolgt.

12. Verfahren nach den Ansprüchen 1—11, dadurch gekennzeichnet, daß die Inkubation unter anaeroben Bedingungen erfolgt.

**Revendications pour les Etats contractants: AT BE LU NL**

1. Procédé microbioligique pour produire la L(−)-carnitine, caractérisé en ce qu'il comprend:

(a) la préparation d'un milieu de culture bactérienne comprenant un composé insaturé choisi parmi la crotonobétaïne, un dérivé de crotonobétaïne;

(b) l'inoculation du milieu de culture avec un microorganisme capable d'hydrater de façon stéréosélective ce composé insaturé en L(−)-carnitine;

(c) l'incubation; et

(d) la récupération de la L(−)-carnitine à partir du milieu de culture.

2. Procédé suivant la revendication 1, caractérisé en ce que le microorganisme est un membre d'un moins une souche des genres Escherichia sp. coli; Salmonella; Shigella; Citrobacter; Clostridium; Hafnia et Proteus.

3. Procédé suivant la revendication 2, caractérisé en ce que la souche du genre Escherichia sp. coli est choisie parmi E. Coli 044 K 74; 055 K 59; 0111 K 58 et 0114 K 90.

4. Procédé suivant la revendication 2, caractérisé en ce que la souche du genre Salmonella est choisie parmi S. typhimurium LT2; S. cottbus; S. anatum et S. newington.

5. Procédé suivant la revendication 2, caractérisé en ce que la souche du genre Shigella est S. flexneri 1a.

6. Procédé suivant la revendication 2, caractérisé en ce que la souche du genre Citrobacter est C. freundii.

7. Procédé suivant la revendication 2, caractérisé en ce que la souche du genre Clostrodium est choisie parmi C. sporogenes et C. kluyveri.

8. Procédé suivant la revendication 2, caractérisé en ce que la souche du genre Hafnia est choisie parmi H. alvei A et H. alvei B..

9. Procédé suivant la revendication 2, caractérisé en ce que la souche du genre Proteus est choisie parmi P. vulgaris et P. mirabilis.

10. Procédé suivant l'une quelconque des revendications 1 à 9, caractérisé en ce que le dérivé de crotonobétaïne est choisi dans le groupe consitant en sels de crotonobétaïne, crotonobetaïne nitrile, crotonobetaïne amides et esters de crotonobetaïne ou leurs mélanges.

11. Procédé suivant l'une quelconque des revendications 1 à 10, caractérisé en ce que la concentration du composé insaturé dans le milieu de culture est comprise entre 10 µmoles et 5 moles/litre.

12. Procédé suivant l'une quelconque des revendications 1 à 11, caractérisé en ce que le milieu de culture est choisi parmi un milieu de culture complexe, un milieu de culture minimum et une solution aqueuse tamponnée à pH 7—7 contenant ce composé insaturé en tant qu'unique source de carbone et d'azote capable d'être métabilisée par ce microorganisme.

13. Procédé suivant la revendication 12, caractérisé en ce que le milieu de réaction comprend un inhibiteur de la réduction du composé insaturé en composé saturé correspondant, choisi parmi des accepteurs d'électron de la respiration aérobie ou anaérobie et des accepteurs d'hydrogène.

14. Procédé suivant la revendication 13, caractérisé en ce que l'inhibiteur est choisi dans le groupe comprenant des nitrates, le triméthylamine N-oxyde et d'autres N-oxydes, le glucose, le fructose, le saccharose, le maltose, le diméthylsulfoxyde, un fumarate, un crotonate et un acrylate.

15. Procédé suivant l'une quelconque des revendications 1 à 14, caractérisé en ce que le microorganisme est un microorganisme qui se développe.

16. Procédé suivant l'une quelconque des revendications 1 à 14, caractérisé en ce que le microorganisme est un microorganisme au repos.

17. Procédé suivant la revendication 15, caractérisé en ce que le microorganisme a été préalablement soumis à une induction.

18. Procédé suivant la revendication 17, caractérisé en ce que l'induction a été effectuée par addition au milieu de culture d'un agent inducteur choisi parmi la crotonoylbétaïne, des esters carboxyliques de L(−), (D+), DL-carnitine, des dérivés O-alkanoyle et des esters de dérivés O-alkanoyle de L(−), (D+), DL-carnitine et leurs sels.

19. Procédé suivant l'une quelconque des revendications 1 à 18, caractérisé en ce que la séparation de la L(−)-carnitine obtenue à partir de la crotonoylbétaïne n'ayant pas réagi, a lieu par conversion de la L(−)-carnitine en une O-alkanoyl carnitine dans laquelle le groupe alkanoyle a de 10 à 18 atomes de carbone et extraction à partir d'une phase aqueuse contenant l'O-alkanoyl carnitine et la crotonoylbétaïne, de l'O-alkanoyl carnitine dnas un alcanol immiscible à l'eau choisi parmi le n-butanol et l'isobutanol.

20. Procédé suivant l'une quelconque des revendications 1 à 19, caractérisé en ce que l'incubation a lieu dans des conditions anaérobies.

**Revendications pour les Etats contractants: CH GB LI**

1. Procédé microbioligique pour produire la L(−)-carnitine, qui comprend:

(a) la préparation d'un milieu de culture bactrienne comprenant un composé insaturé choisi parmi la crotonobétaïne, un dérivé de crotonobétaïne;

(b) l'inoculation du milieu de culture avec un microorganisme capable d'hydrater de façon stéréosélective ce composé insaturé en L(−)-carnitine;

(c) l'incubation du microorganisme; et

(d) la récupération de la L(−)-carnitine à partir du milieu de culture, caractérisé en ce que le miroorganisme est choisi dans le groupe consistant en:

Escherichia Coli 044 K 74
Escherichia Coli 055 K 59
Escherichia Coli 0111 K 58
Escherichia Coli 0114 K 90
Salmonella typhimurium LT2
Salmonella S. cottbus
Salmonella S. anatum
Salmonella S. newington
Shigella flexneri
Clostrodium kluyveri
Clostrodium sporogenes
Citrobacter freundii
Proteus vulgaris

Proteus mirabilis
Hafnia alvei.

2. Procédé suivant la revendication 1, caractérisé en ce que le dérivé de crotonobétaïne est choisi dans le groupe consitant en sels de crotonobétaïne, crotonobetaïne nitrile, crotonobetaïne amide et esters alkyliques de crotonobetaïne ou leurs mélanges.

3. Procédé suivant la revendication 1 et 2, caractérisé en ce que la concentration du composé insaturé dans le milieu de culture est comprise entre 10 µmoles et 5 moles/litre.

4. Procédé suivant l'une quelconque des revendications 1 à 3, caractérisé en ce que le milieu de culture est choisi parmi un milieu de culture complexe, un milieu de culture minimum et une solution aqueuse tamponnée à pH 7—8 contenant ce composé insaturé en tant qu'unique source de carbone et d'azote capable d'être métabilisée par ce microorganisme.

5. Procédé suivant la revendication 4, caractérisé en ce que le milieu de réaction comprend un inhibiteur de la réduction du composé insaturé en composé saturé correspondant, choisi parmi des accepteurs d'électron de la respiration aérobie ou anaérobie et des accepteurs d'hydrogène.

6. Procédé suivant la revendication 5, caractérisé en ce que l'inhibiteur est choisi dans le groupe comprenant des nitrates, le triméthylamine N-oxyde et d'autres N-oxydes, le glucose, le fructose, le saccharose, le maltose, le diméthylsulfoxyde, un fumarate, un crotonate et un acrylate.

7. Procédé suivant l'une quelconque des revendications 1 à 6, caractérisé en ce que le microorganisme est un microorganisme qui se développe.

8. Procédé suivant l'une quelconque des revendications 1 à 6, caractérisé en ce que le microorganisme est un microorganisme au repos.

9. Procédé suivant la revendication 7, caractérisé en ce que le microorganisme a été préalablement soumis à une induction.

10. Procédé suivant la revendication 9, caractérisé en ce que l'induction a été effectuée par addition au milieu de culture d'un agent inducteur choisi parmi la crotonoylbétaïne, des esters carboxyliques de L(−), (D+), DL-carnitine, des dérivés O-alkanoyle et des esters de dérivés O-alkanoyle de L(−), (D+), DL-carnitine et leurs sels.

11. Procédé suivant l'une quelconque des revendications 1 à 10, caractérisé en ce que la séparation de la L(−)-carnitine obtenue à partir de la crotonoylbétaïne n'ayant pas réagi, a lieu par conversion de la L(−)-carnitine en une O-alkanoyl carnitine dans laquelle le groupe alkanoyle a de 10 à 18 atomes de carbone et extraction à partir d'une phase aqueuse contenant l'O-alkanoyl carnitine et la crotonoylbétaïne, de l'O-alkanoyl carnitine dans un alcanol immiscible à l'eau choisi parmi le n-butanol et l'isobutanol.

12. Procédé suivant l'une quelconque des revendications 1 à 11, caractérisé en ce que l'incubation a lieu dans des conditions anaérobies.

**Revendications pour les Etats contractants: DE FR IT SE**

1. Procédé microbioligique pour produire la L(−)-carnitine, qui comprend:

(a) la préparation d'un milieu de culture bacfrienne comprenant un composé insaturé choisi parmi la crotonobétaïne, un dérivé de crotonobétaïne;

(b) l'inoculation du milieu de culture avec un microorganisme capable d'hydrater de façon stéréosélective ce composé insaturé en L(−)-carnitine;

(c) l'incubation du microorganisme; et

(d) la récupération de la L(−)-carnbitine à partir du milieu de culture, caractérisé en ce que le miroorganisme est choisi dans le groupe consistant en:
Escherichia Coli 044 K 74
Escherichia Coli 055 K 59
Escherichia Coli 0111 K 58
Escherichia Coli 0114 K 90
Salmonella typhimurium LT2
Salmonella S. cottbus
Salmonella S. anatum
Salmonella S. newington
Shigella flexneri
Clostrodium kluyveri
Clostrodium sporogenes
Citrobacter freundii
Proteus vulgaris

2. Procédé suivant la revendication 1, caractérisé en ce que le dérivé de crotonobétaïne est choisi dans le groupe consitant en crotonobétaïne nitrile, crotonobetaïne amides et esters de crotonobetaïne.

3. Procédé suivant l'une quelconque des revendications 1 et 2, caractérisé en ce que la concentration du composé insaturé dans le milieu de culture est comprise entre 10 µmoles et 5 moles/litre.

4. Procédé suivant l'une quelconque des revendications 1 à 3, caractérisé en ce que le milieu de culture est choisi parmi un milieu de culture complexe, un milieu de culture minimum et une solution aqueuse tamponnée à pH 7—8 contenant ce composé insaturé en tant qu'unique source de carbone et d'azote

capable d'être métabilisée par ce microorganisme.

5. Procédé suivant la revendication 4, caractérisé en ce que le milieu de réaction comprend un inhibiteur de la réduction du composé insaturé en composé saturé correspondant, choisi parmi des accepteurs d'électron de la respiration aérobie ou anaérobie et des accepteurs d'hydrogène.

6. Procédé suivant la revendication 5, caractérisé en ce que l'inhibiteur est choisi dans le groupe comprenant des nitrates, le triméthylamine N-oxyde et d'autres N-oxydes, le glucose, le fructose, le saccharose, le maltose, le diméthylsulfoxyde, un fumarate, un crotonate et un acrylate.

7. Procédé suivant l'une quelconque des revendications 1 à 6, caractérisé en ce que le microorganisme est un microorganisme qui se développe.

8. Procédé suivant l'une quelconque des revendications 1 à 6, caractérisé en ce que le microorganisme est un microorganisme au repos.

9. Procédé suivant la revendication 7, caractérisé en ce que le microorganisme a été préalablement soumis à une induction.

10. Procédé suivant la revendication 9, caractérisé en ce que l'induction a été effectuée par addition au milieu de culture d'un agent inducteur choisi parmi la crotonoylbétaïne, des esters carboxyliques de L(−), (D+), DL-carnitine, des dérivés O-alkanoyle et des esters de dérivés O-alkanoyle de L(−), (D+), DL-carnitine et leurs sels.

11. Procédé suivant l'une quelconque des revendications 1 à 10, caractérisé en ce que la séparation de la L(−)-carnitine obtenue à partir de la crotonoylbétaïne n'ayant pas réagi, a lieu par conversion de la L(−)-carnitine en une O-alkanoyl carnitine dans laquelle le groupe alkanoyle a de 10 à 18 atomes de carbone et extraction à partir d'une phase aqueuse contenant l'O-alkanoyl carnitine et la crotonoylbétaïne, de l'O-alkanoyl carnitine dans un alcanol immiscible à l'eau choisi parmi le n-butanol et l'isobutanol.

12. Procédé suivant l'une quelconque des revendications 1 à 11, caractérisé en ce que l'incubation a lieu dans des conditions anaérobies.